# EUROPEAN PATENT APPLICATION

(11) **EP 1 142 474 A1**
(43) Date of publication of application: **10.10.2001**
(21) Application number: 01107395.4
(22) Date of filing: 26.03.2001
(51) Int. Cl.: A01N 3/02, A01N 37/44, C07C 323/58

(54) **Cut flower anti-senescent preservative**

(30) Priority: 05.04.2000 JP 2000103957
(71) Applicant: Hasunuma, Kohij, Machida City, Tokyo, 194-0004 (JP); Palace Chemical Co., Ltd., Kanagawa, 236-0004 (JP)
(72) Inventor: Yabe, Naoto, Kihara Inst. f. Biological Research, Yokohama City, Kanagawa, 244-0813 (JP)
(74) Representative: Hiltl, Elmar, Dr.

(57) **Abstract**

A cut flower anti-senescent preservative containing at least one L-isomer of the compounds represented by the formula HOOC-CH(NH₂)-CH₂-CH₂-S-R wherein R represents a lower alkyl group having two or more carbon atoms, or containing at least one of the compounds represented by said formula and a detergent. The anti-senescent preservative of the invention has an excellent effect for cut flowers, and is ecologically benign.

## Description

### FIELD OF THE INVENTION

The invention relates to a cut flower anti-senescent preservative.

### DESCRIPTION OF THE RELATED ART

Longevity of cut flowers is decreased by the factors such as rot (bacterial contamination) of a peduncle, chalk of vascular systems in water, starvation of nutrients and senescence induced by endogenous and exogenous ethylene. There are used bactericides such as 8-hydroxyquinoline to prevent rot of a peduncle or chalk of vascular systems , detergents for improving water uptake, and sugars such as sucrose for starvation of nutrients. With respect to ethylene, silver thiosulfate (STS) found by Veen et al., Netherlands in 1978 [Planta, vol. 40, pp. 93 - 96 (1978)] inhibits action of ethylene, and is clearly effective for longevity of cut flowers of *Dianthus caryophyllus* (carnation) and *Gypsophila paniculata* L. which cause petal shrinkage mainly by ethylene. Many flower makers are currently using STS. However, since silver thiosulfate contains silver, aheavymetal, it could be problematic in environmental pollution. Further, it is reported that aminooxyacetic acid (AOA) alone keeps longevity of carnation [Hort Science, vol. 20, pp. 41 - 45 (1985)], but it is not effective for other cut flowers of *Lathyrus odoratus* (sweet pea) and *Delphinium ajacis* L. (rocket larkspur) [Engakushi 62 betsu 2, pp. 442- 443 (1993)].

### SUMMARY OF THE INVENTION

The invention aims to provide an anti-senescent preservative which has an excellent effect on cut flowers and is ecologically harmless because of the absence of heavy metals .

The cut flower anti-senescent preservative of the invention is characterized by containing at least one L-isomer of the compounds represented by the formula

HOOC-CH(NH₂)-CH₂-CH₂-S-R

wherein R represents a lower alkyl group having two or more carbon atoms,
or containing at least one of the compounds represented by the formula and a detergent.

### BRIEF DESCRIPTION OF THE DRAWING

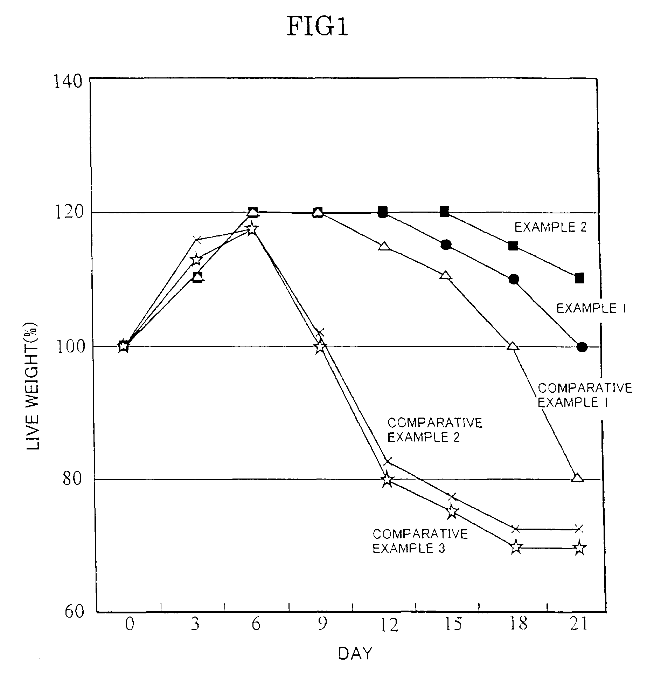

Fig. 1 is a graph showing the change of the fresh weight during a test for longevity of flower.

### DETAILED DESCRIPTION OF THE INVENTION

In the formula, R is a lower alkyl group having two or more carbon atoms, such as ethyl, propyl or butyl. Among the compounds represented by the formula, ethionine

HOOC-CH(NH₂)-CH₂-CH₂-S-CH₂-CH₃

can easily be procured in the market.
The anti-senescent preservative of the present invention is usually used as an aqueous solution. In use, the concentration of the compound represented by the formula is not particularly limited because its optimum value varies depending on species of cut flowers. It is generally present in an amount of between 0.001 and 10 wt-%, preferably between 0.01 and 1 wt-% based on the weight of the aqueous solution.

The cut flower anti-senescent preservative of the invention can usually be formulated into various types such as an emulsion, a water-dispersible powder, a flowable composition, an aqueous solution , a sheet, a granule and a tablet by mixing the compound represented by the formula with a solid carrier, a liquid carrier, a detergent and other auxiliaries. Usually, it is advisable that highly concentrated products are diluted with water in use.

Examples of the solid carrier include fine powders or particles of kaolin, clay, attapulgite clay, bentonite, acid clay, pyrophyllite, talc, diatomaceous earth, calcite, walnut powder, urea, ammonium sulfate and synthetic hydrous silicon oxide. Examples of the liquid carrier include aromatic hydrocarbons such as xylene and methylnaphthalene, alcohols such as ethanol, isopropanol, ethylene glycol and cellosolve, ketones such as acetone and cyclohexane, vegetable oils such as soybean oil and cottonseed oil, dimethyl sulfoxide, acetonitrile and water.

Examples of the detergent include anionic detergents such as an alkylbenzene sulfonate , an alkyl sulfate , a higher alcohol sulfate, an alkylaryl sulfonate, a dialkyl sulfosuccinate and a polyoxyethylene alkylaryl ether phosphate, and nonionic detergents such as a polyoxyethylene alkyl ether, a polyoxyethylene alkylaryl ether, a polyoxyethylene polyoxypropylene block copolymer, a sorbitan fatty acid ester and a sucrose fatty acid ester. When the detergent is added to the compound represented by the formula, the longevity preservation effect can be increased by improving the water uptake of cut flowers and increasing efficiency of incorporation into plant cells. It is advisable that the amount of the detergent is adjusted such that the concentration thereof in an aqueous solution during use is between 0.001 and 1.0 %.

Examples of auxiliaries include lignin sulfonate, an alginate, polyvinyl alcohol, gum arabic, CMC (carboxymethyl cellulose) and PAP (acidic isopropyl phosphate).

The cut flower anti-senescent preservative of the invention can contain another anti-senescent preservative, an antiseptic, a bactericide, sugars as a nutrient source, a water uptake promoter, a phytohormone for prevention of yellowing of fruits, a fertilizer ingredient and an insecticide unless the effect of the invention is impaired. Examples of another anti-senescent preservative include aminooxyacetic acid (AOA), 1-a-(2-aminoethoxyvinyl)glycine (AVG) and 2-aminoisobutyric acid (AIB). Examples of the bactericide include 8-hydroquinoline sulfate or citrate, chlorine-containing compounds such as sodium hypochlorite and sodium dichloroisocyanurate, quaternary ammonium salts such as benzalkonium chloride, dehydroacetic acid and its salts, benzoic acid and its salts , sorbic acid and its salts . Examples of the sugars include glucose, fructose, sucrose, sorbitol, maltose, trehalose and sorbitol. Examples of the water uptake promoter include aluminum sulfate and alum. Examples of the phytohormone include cytokinins such as benzylaminopurine and kinetin, gibberellins, abscisic acid, auxins and brassinosteroids. Examples of the fertilizer ingredient include urea and ammonium nitrate containing nitrogen, potassium phosphate and ammonium phosphate containing phosphate, and potassium sulfate and potassium hydrochloride containing potassium. Examples of the insecticide include pyrethroid insecticides such as permethrin and phenothrin, organophosphorus insecticides such as fenitrothion and fenthion, carbamate insecticides such as carbaryl. IGR and a chitin synthesis inhibitor can be contained further.

Examples of cut flowers to which the anti-senescent preservative of the invention can apply include carnation, sweet pea, *Grysophila elegans* Bieb., *Gypsophila paniculata* L., lily, *Delphinium ajacis* L. orchids, *Matthiola incana* R. Br. (stock), *Limonium* (sea lavender), *Limonium hybrid* (statis), gladiolus (sword lily), tulip and rose.

The anti-senescent preservative of the invention is used by dipping a cut portion of a cut flower in a solution containing the anti-senescent preservative. At this time, it may be dipped in the anti-senescent preservative all the time or may be dipped in the anti-senescent preservative for a fixed period of time and then put in water. In the latter case, it is advisable that the time for dipping in the anti-senescent preservative is between 1 and 72 hours . Alternatively, a solution containing the anti-senescent preservative may be sprayed on leaves or petals of a cut flower. Incidentally, another anti-senescent preservative can be added, as required, unless the effect of the active ingredient is impaired.

Among the conventional cut flower anti-senescent preservatives, STS (silver thiosulfate anionic complex) aimed at inhibition of reception mechanism of ethylene (a phytohormone which is the main factor responsible for senescence of a cut flower), and aminooxyacetic acid (AOA) and aminoethoxyvinylglycine (AVG) aimed at inhibition of enzymes in ethylene biosynthesis pathway such as ACS (1-aminocyclopropane-1-carboxylate synthase) and ACO (1-aminocyclopropane-1-carboxylate oxidase).

However, it is indicated that since STS contains silver, a heavy metal, as a main ingredient, it is problematic in environmental pollution and disposal of waste solution after treatment causes high costs.

Further, it is demonstrated that AOA is effective for maintaining longevity of carnation but not effective for other flowers. This is presumably because it targets a specific enzyme and there is a difference in level of sensitivity to an inhibitor among specific plants.

In the invention, a specific enzyme is not a target. By incorporating an analogue of methionine (a precursor of ethylene biosynthesis), the precursor is diluted in the reaction system, to decrease the absolute amount of generated ethylene (phytohormone responsible for senescence of cut flower), whereby senescence of cut flower is restrained.

L-ethionine or its derivatives (though some of them does not exist in the natural world) are biodegradable amino acids, and do not cause a problem of environmental pollution as caused by STS etc., and disposal of waste liquor is easily conducted.

### EXAMPLES

The invention is illustrated specifically by referring to the following Examples. However, the invention is not limited to these Examples.

### Example 1

An aqueous solution containing 0.016% of L-ethionine was prepared.

### Example 2

An aqueous solution containing 0.016 % of L-ethionine, 0.02 % of polyoxysorbitan fatty acid ester and 0.05 % of citric acid was prepared.

### Comparative Example 1

An aqueous solution containing 0.07 % of silver thiosulfate was prepared.

### Comparative Example 2

An aqueous solution containing 0.016 % of L-methionine was prepared.

### Comparative Example 3

Tap water was used.

### Test Example 1

Ten untreated cut flowers of carnation were dipped in each of the solutions in Examples and Comparative Examples for 5 hours , and then put in tap water. These flowers were compared with respect to a period for longevity of flower.

The number of days for longevity of carnation was measured in terms of the number of days that lapsed until six of the ten cut flowers withered. The test results are shown in Table 1.

**Table 1**

| Specimen | Ingredient | Number of days for longevity of flower |
|---|---|---|
| Example 1 | L-ethionine 0.016 % | 18 |
| Example 2 | L-ethionine 0.016 + polyoxysorbitan fatty acid ester 0.02 % + citric acid 0.015 % | 21 |
| Comparative Example 1 | silver thiosulfate 0.07 % | 21 |
| Comparative Example 2 | L-methionine 0.016 % | 6 |
| Comparative Example 3 | city water | 7 |

As is clear from Table 1, the cut flower anti-senescent preservatives in Examples 1 and 2 show approximately the same number of days for longevity of flower as the known silver thiosulfate.

### Test Example 2

With respect to the cut flowers of carnation during the test in Test Example 1, the fresh weight (total value of ten cut flowers in Examples and Comparative Examples) was measured on days 3, 6, 9, 12, 15, 18 and 21 after the start-up of the test. The results are shown in Fig. 1 in terms of indexes in which the fresh weight in the start-up of the test was rated as 100.

As is apparent from Fig. 1, the cut flower anti-senescent preservatives in Examples 1 and 2 are good in water uptake and excellent as compared with the known silver thiosulfate.

Thus, the invention provides the anti-senescent preservatives which exhibit the excellent effect for cut flowers and are ecologically harmless.

## Claims

1. A cut flower anti-senescent preservative containing at least one L-isomer of the compounds represented by the formula
HOOC-CH(NH₂)-CH₂-CH₂-S-R
wherein R represents a lower alkyl group having two or more carbon atoms.

2. A cut flower anti-senescent preservative containing at least one of the compounds represented by the formula
HOOC-CH(NH₂)-CH₂-CH₂-S-R
wherein R represents a lower alkyl group having two or more carbon atoms
and a detergent.

3. The cut flower anti-senescent preservative as claimed in claim 1 or 2, wherein R is an ethyl group.
